## Europäisches Patentamt
## European Patent Office
## Office européen des brevets

(11) Veröffentlichungsnummer: **0 387 760**
A1

# EUROPÄISCHE PATENTANMELDUNG

(21) Anmeldenummer: 90104652.4

(22) Anmeldetag: 12.03.90

(51) Int. Cl.5: **C12N 9/00, C07K 3/12**

(30) Priorität: 15.03.89 DE 3908422

(43) Veröffentlichungstag der Anmeldung:
19.09.90 Patentblatt 90/38

(84) Benannte Vertragsstaaten:
AT BE CH DE DK FR GB IT LI NL SE

(71) Anmelder: **Forschungszentrum Jülich GmbH
Postfach 1913
D-5170 Jülich(DE)**

(72) Erfinder: **Kula, Maria Regina, Prof., Dr.
Selgenbusch 12
D-5162 Niederzier(DE)**
Erfinder: **Vernau, Jörg
Prämienstrasse 21
D-5170 Jülich(DE)**

(54) **Verfahren zur Extraktion von Proteinen aus wässrigen Biomassesuspensionen.**

(57) Zur Extraktion von Proteinen, insbesondere Enzymen, aus wäßrige Biomassesuspensionen durch Phasenverteilung mittels eines wässrigen Polymer/Salz-Zweiphasensystems eignet sich als Salz - vor allem aus Gründen der biologischen Entsorgung -lösliches Citrat. Als Citrat sind Natrium- und/oder Kaliumcitrat ggf. in Mischung mit Citronensäure geeignet. Verwendet werden speziell Mischungen von Natrium-und/oder Kaliumcitrat mit Citronensäure mit pH-Werten von 4 bis 10, insbesondere von 6 bis 9. Als Polymerkomponente eignet sich Polyethylenglykol mit einem Molekulargewicht von 400 bis 20 000, insbesondere von 1000 bis 4000. Da prinzipiell keine Temperaturbeschränkungen bestehen, kann die Extraktion bei Zimmertemperatur durchgeführt werden. Das in die Oberphase extrahierte Protein bzw. Enzym wird mittels eines weiteren 2-Phasensystems in eine salzhaltige Unterphase unter Verwendung von NaCl reextrahiert.

PEG 1550 - Na³ Citrat pH 7.0

FIG. 2

EP 0 387 760 A1

# Verfahren zur Extraktion von Proteinen aus wäßrigen Biomassesuspensionen

Die Erfindung bezieht sich auf ein Verfahren zur Extraktion von Proteinen, insbesondere Enzymen, aus wäßrigen Biomasse-Suspensionen durch Phasenverteilung mittels eines wäßrigen Polymer/Salz-Zweiphasensystems.

Wäßrige Phasensysteme entstehen aus der sogenannten Unverträglichkeit von unterschiedlichen Polymeren in Wasser als gemeinsamem Lösungsmittel oder durch das Aussalzen von hydrophilen Polymeren wie Polyethylenglykol (PEG) aus Wasser. Für letztere Systeme sind Kalium- und Natriumphosphat sowie Ammonium- bzw. Magnesiumsulfat als Salzkomponente bekannt (s. P.A. Albertsson, Partition of Cell Particles and Macromolecules, 3rd Edition, Wiley, New York, 1986).

Solche Systeme können erfolgreich zur Abtrennung von Proteinen wie insbesondere Enzymen (Zielprotein) aus biomassehaltigen Suspensionen wie insbesondere Zellaufschlußmassen verwendet werden: Das Zielprotein sammelt sich in der polymerhaltigen Oberphase und kann auf diese Weise sehr elegant von Zellbruchstücken abgetrennt werden, die sich in der salzhaltigen Unterphase anreichern (siehe DE-PS 26 39 129 und DE-OS 29 43 016).

Die Effizienz dieser wäßrigen Phasensysteme zur Extraktion von Proteinen aus biologischen Rohstoffen ist unbestritten.

Für großtechnische Anwendungen werden z.Zt. Kreisläufe für die Hilfschemikalien zur Phasenbildung gesucht, um Rohstoffkosten zu senken (N. Papamichael, H. Hustedt Zur Wirtschaftlichkeit der Flüssigextraktion von Enzymen, Biotech Forum 4, 259-69, 1987) . Darüber hinaus sollte die Salzfracht der Abläufe aber auch aus Gründen des Umweltschutzes deutlich gesenkt werden. Dies gilt insbesondere für PEG/Phosphat- und PEG/Sulfat-Systeme.

Ziel der Erfindung ist daher ein Extraktionsverfahren der eingangs genannten Art, bei dem die Umweltbelastung durch Abläufe merklich vermindert werden kann.

Das zu diesem Zweck entwickelte erfindungsgemäße Verfahren ist dadurch gekennzeichnet, daß man als Salz für zu verwerfende Unterphase Natrium-, Kalium- und/oder Ammoniumcitrat/Citronensäure-Mischungen mit pH 6 bis 9 verwendet.

Es wurde nämlich gefunden, daß bei der wäßrigen 2-Phasen-Extraktion von Proteinen vergleichbar gute Ergebnisse erzielt werden, wenn man anstelle der Phosphate bzw. Sulfate Citrat verwendet, das biologisch vollständig abgebaut werden kann und toxikologisch unbedenklich ist.

Gemäß der Erfindung ist insbesondere die in einer ersten Extraktionsstufe anfallende Unterphase, welche die Feststoffanteile der eingesetzten Biomasse enthält und sehr schwer weiter entwässert und konditioniert werden kann, einer vollständigen biologischen Entsor gung zugänglich. Aber auch in späteren Stufen anfallende Prozeßabwässer sind bei Verwendung von Citrat wesentlich einfacher zu entsorgen.

Als Citrat sind insbesondere neben Ammoniumcitrat Natrium-und/oder Kaliumcitrat ggf. in Mischung mit Citronensäure geeignet.

Verwendet werden speziell Mischungen von Natrium-und/oder Kaliumcitrat mit Gitronensäure mit pH-Werten von 4 bis 10, insbesondere 6 bis 9. Zur pH-Einstellung kann selbstverständlich auch andere Säure (als Citronensäure) verwendet werden.

Als Polymerkomponente sind ganz allgemein lösliche hydrophile Polymere, insbesondere Derivate von Polyethern, anwendbar. Besonders zweckmäßig, da verfügbar und umfänglich untersucht, sind Polyethylenglykole mit einem Molekulargewicht von 400 bis 20000, insbesondere 1000 bis 4000.

Prinzipiell bestehen keine Temperaturbeschränkungen, so daß z.B. sonst übliche Temperaturen von 10 bis 30°C ohne weiteres gewählt werden können.

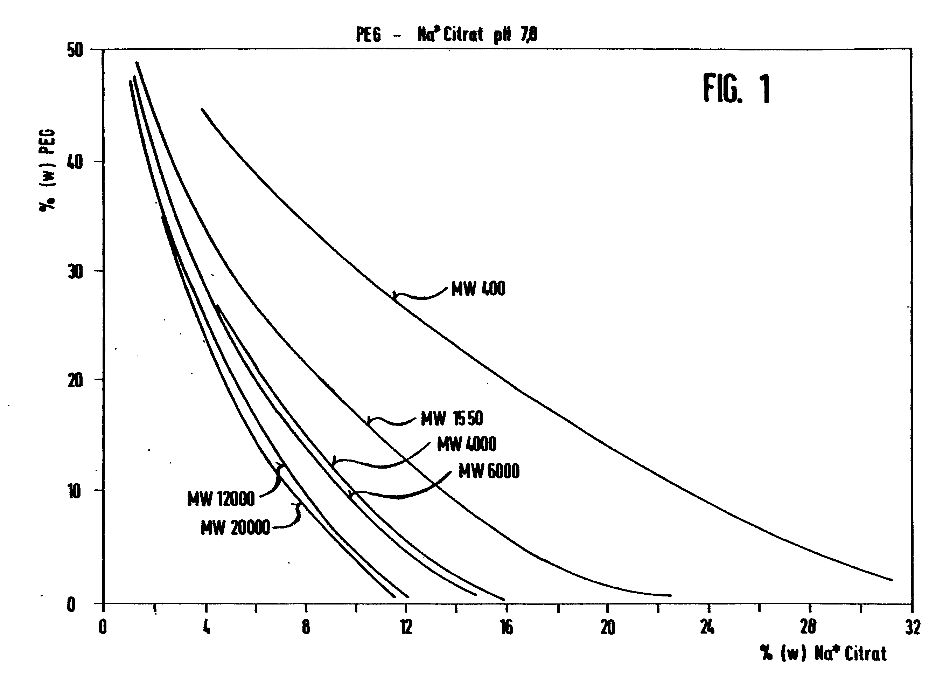

Die brauchbaren Arbeitsbereiche ergeben sich anhand von Binodalen und Konoden. Als Beispiel sind in der beigefügten Figur 1 die Binodalen für das System Polyethylenglykol/Natriumcitrat-Citronensäure von pH 7,0/Wasser für Polyethylenglykole mit unterschiedlichen Molekulargewichten aufgetragen. Links der Binodalen befindet sich die Lösung im Einphasengebiet, die dann durch Konzentrationserhöhung des Polymers und/oder des Salzes die Binodale und somit das 2-Phasen-Gebiet erreicht und sich entmischt.

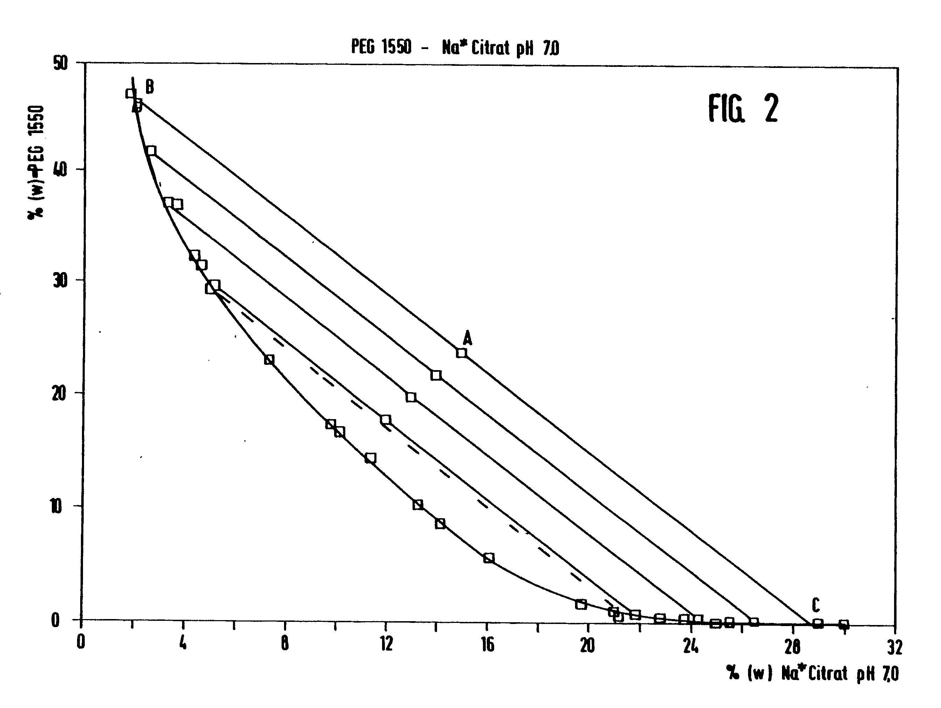

Figur 2 zeigt die Binodale für das System PEG 1550/Natriumcitrat-Citronensäure von pH 7,0/Wasser sowie die zugehörigen Konoden. Wird eine Lösung mit Komponenten wie z.B. in Punkt A erstellt, so zerfällt diese in die Oberphase mit den Konzentrationen an PEG und Na-Citrat am Punkt B bzw. die Unterphase mit der Zusammensetzung an Punkt C. Das Verhältnis zu $\overline{AC}$ zu $\overline{AB}$ gibt das Massenverhältnis von Oberphase zu Unterphase wieder. Somit läßt sich durch Verschieben des Punktes A das Gewichts- und Volumenverhältnis der beiden Phasen variieren. Damit hat man z.B. die Möglichkeit, durch Volumenvergrößerung der Phase mit dem Zielprotein, dessen Ausbeute bei nicht optimalem Verteilungskoeffizienten zu steigern. Andererseits kann man z.B. bei sehr hohem Verteilungskoeffizienten des Zielproteins das Volumen der weiterzuverarbeitenden Phase geringhalten und dadurch die Konzentration des Zielproteins erhöhen.

Die Kurven wurden bei Zimmertemperatur aufgenommen. Mit Kaliumcitrat- bzw. Kalium/Natriumcitrat-Citronensäure-Mischungen von pH 7 erhält man ähnliche Diagramme.

Die Binodalkurven stecken den brauchbaren Arbeitsbereich ab: So kann z.8. bei Zimmertemperatur mit PEG/Na-Citrat-Mischungen gearbeitet werden, die 1 -40 Gew% PEG bzw. 1 - 30 Gew% Na-Citrat pH 7,0 enthalten. Die Konoden ergeben dann Zusammensetzung und Anteile der sich bildenden Phasen.

Für die praktische Anwendung wird man üblicherweise ausgehend von mittleren Konzentrationen PEG- bzw. Salz-Verbrauchsoptimierungen vornehmen unter Berück sichtigung, daß durch die Anwesenheit von Biomasse eine gewisse Verschiebung der Mischbarkeit zum Nullpunkt hin auftritt.

Bei der Extraktion geht das Protein bzw. Enzym fast vollständig in die PEG-haltige Oberphase und wird somit von den mit konventionellen Methoden nur schwer abtrennbaren Feststoffen (wie z.B. Zellbruchstük-ken) abgetrennt. Aus dieser PEG-haltigen Oberphase wird das extrahierte Protein (insbesondere Enzym) zweckmäßigerweise in einem zweiten Schritt mittels eines weiteren 2-Phasensystems in eine salzhaltige Unterphase reextrahiert, wodurch zum einen weitere Reinigungsschritte erleichtert und zum anderen die abgereicherte PEG-Phase rezykliert werden kann bzw. können.

Bei diesem Reextraktionsschritt wird der Verteilungskoeffizient des Proteins z.B. durch veränderten Salzgehalt und/oder pH-Wert des Systems dahingehend beeinflußt, daß sich das Zielprotein in der Unterphase anreichert. Dies kann z.B. durch Zugabe von NaCl in Konzentrationen von $\geq$ 50 mmol/l unterstützt werden.

Nachfolgend wird die Erfindung anhand praktischer Beispiele erläutert:


Beispiel 1

Zellen von Bäckerhefe (Saccharomyces cerevisiae) wurden mechanisch aufgeschlossen (Partikelgröße bis 5 µm). Der 40% Zellfeuchtmasse enthaltende Zellaufschluß wurde dann zur Gewinnung der Enzyme Alkoholdehydrogenase (ADH) und Glucose-6-Phosphatdehydrogenase (G-6-PDH) einer Extraktion mittels eines wäßrigen 2- Phasen-Systems mit PEG 4000 und Na-Citrat/Citronensäure von pH 8,5 unterworfen. Die Zusammensetzung des Gesamtsystems ist in Tabelle 1 angegeben.

Bei der ersten Extraktion geht das Enzym in die PEG-haltige Oberphase. Aus dieser Oberphase werden die Enzyme rückextrahiert, wobei die Rückextraktion des Enzyms in die salzhaltige Unterphase durch leicht erhöhte Salzkonzentration und geänderten pH-Wert unterstützt wird.


Beispiel 2

Die Verfahrensweise von Beispiel 1 wurde wiederholt, jedoch wurden anstelle von Natriumcitrat/Citronensäure Kaliumcitrat/Citronensäure und anstelle von PEG 4000 PEG 1000 für die erste Extraktion und ein Zusatz von PEG 12000 für die Rückextraktion vorgesehen. Die Zusammensetzungen sind in Tabelle 1 angegeben.


Beispiel 3

Die Verfahrensweise von Beispiel 2 wurde wiederholt, jedoch wurde Natriumcitrat/Citronensäure anstelle von Kaliumcitrat/Citronensäure verwendet. Die Zusammensetzungen sind in Tabelle 1 wiedergegeben.

Die jeweils erzielten Ergebnisse sind in Tabelle 2 zusammengefaßt.


Beispiel 4

Aufgeschlossene Zellen von Bacillus cereus wurden zur Gewinnung der Enzyme Leucindehydrogenase (Leu DH) und Alanindehydrogenase (AlaDH) einer wäßrigen 2-Phasen-Extraktion unter Verwendung von PEG 1550 und einer Mischung von Natriumcitrat und Citronensäure von pH 7,0 in den in Tabelle 1 angegebenen Mengenverhältnissen unterworfen, wobei die Enzyme sich in der PEG-haltigen Oberphase sammeln.

Zur Rückextraktion der Enzyme wurde erneut eine Phasenverteilung mit der in Tabelle 1 angegebenen Mischung vorgenommen: In diesem Falle wird die Rückextraktion des Enzyms in die salzhaltige Unterphase durch den Zusatz von NaCl unterstützt.


Beispiel 5

Aus Mandelkleie wurde das Enzym R-Oxynitrilase durch wäßrige Phasen-Verteilung unter Anwendung der in Tabelle 1 angegebenen Mischungen extrahiert.

Aus der PEG-haltigen Oberphase der ersten Extraktion wird die R-Oxynitrilase in einer zweiten Stufe unter Zusatz von NaCl und einer Gesamtzusammensetzung, wie in Tabelle 1 angegeben, rückextrahiert.

In Tabelle 2 sind die in den Beispielen 1 bis 5 benutzten Systeme und erzielten Ergebnisse zusammengefaßt.


Tabelle 1:

Phasensysteme für (ADH/G-6-PDH) aus Bäckerhefe

      1) I. 50 % w Zellaufschluß (40 % Zellfeuchtmasse)
8 % w Na-Citrat/Gitronensäure pH 8,5
7,5 % w PEG 4000
34,5 % w $H_2O$ (zusätzlich)
II. 50 % w Oberphase I
11 % w Na-Citrat/Citronensäure pH 7,0
39 % w $H_2O$ (zusätzlich)
      2) I. 50 % w Zellaufschluß (40 % Zellfeuchtmasse)
9 % w K-Citrat/Citronensäure pH 8,5
17 % w PEG 1000
24 % w $H_2O$ (zusätzlich)
II. 50 % w Oberphase I
11 % w K-Citrat/Citronensäure pH 8,5
0,5 % w PEG 12000
38,5 % w $H_2O$ (zusätzlich)
      3) I. 50 % w Zellaufschluß (40 % Zellfeuchtmasse)
9 % w Na-Citrat/Citronensäure pH 8,5
17 % w PEG 1000
24 % w $H_2O$ (zusätzlich)
II. 50 % w Oberphase I
11 % w Na-Citrat/Citronensäure pH 8,5
0,5 % w PEG 12000
38,5 % w $H_2O$ (zusätzlich)


Phasensysteme für (LeuDH/AlaDH) aus Bacillus cereus

      4) I. 50 % w Zellaufschluß (40 % Zellfeuchtmasse)
19 % w PEG 1550
2,2 % w Na-Citrat/Citronensäure pH 7,0
28,8 % w $H_2O$ (zusätzlich)
II. 50 % w Oberphase I
11 % w Na-Citrat/Citronensäure pH 7,0
5,6 % w NaCl
33,4 % w $H_2O$ (zusätzlich)


Phasensysteme für (R-Oxynitrilase) aus Mandelkleie

      5) I. 10 % w Mandelkleie
21 % w PEG 1550

4

6,25 % w Na-Citrat/Citronensäure pH 7,0
62,75 % w $H_2O$
II. 50 % w Oberphase I
9 % w Na-Citrat/Citronensäure pH 7,0
1,91 % w NaCl
39,09 % w $H_2O$ (zusätzlich)

Tabelle 2:

| Beispiel | Enzym | Biomasse | Biomassekonzentr.% | Phasensystem | Enzymausbeute % | Anreicherung |
|---|---|---|---|---|---|---|
| 1 | I. ADH/G-6-PDH | Bäckerhefe | 20 % w feucht | PEG/Na-Citrat | 84/81 | 1,5/1,7 |
|   | II. | Oberphase I |   | PEG/Na-Citrat | 99/98 | 1,6/1,8 |
| 2 | I. ADH/G-6-PDH | Bäckerhefe | 20 % w feucht | PEG/K-Citrat | 89/83 | 1,3/1,4 |
|   | II. | Oberphase I |   | PEG/K-Citrat | 99/99 | 1,5/1,6 |
| 3 | I. ADH/G-6-PDH | Bäckerhefe | 20 % w feucht | PEG/Na-Citrat | 89/87 | 1,4/1,4 |
|   | II. | Oberphase I |   | PEG/Na-Citrat | 99/99 | 1,5/1,7 |
| 4 | I. LeuDH/AlaDH | Bacillus cereus | 20 % w feucht | PEG/Na-Citrat | 84/59 | 3,9/2,0 |
|   | II. | Oberphase I |   | PEG/Na-Citrat | 85/85 | 4,0/2,0 |
| 5 | R-Oxynitrilase | Mandelkleie | 10% w trocken | PEG/Na-Citrat | 99 | 14,6 |
|   | II. | Oberphase I |   | PEG/Na-Citrat | 86 | 17,5 |

I. = Extraktion des Enzyms aus der Biomasse in die PEG-reiche Oberphase (1. Phasensystem)

II. = Extraktion des Enzyms aus der PEG-reichen Phase in die salzreiche Unterphase (2. Phasensystem)

## Ansprüche

1. Verfahren zur Extraktion von Proteinen, insbesondere Enzymen, aus wäßrigen Biomasse-Suspensionen durch Phasenverteilung mittels eines wäßrigen Polymer/Salz-Zweiphasensystems,
**dadurch gekennzeichnet,**
daß man als Salz für zu verwerfende Unterphasen Natrium-, Kalium-und/oder Ammoniumcitrat/Citronensäure-Mischungen mit pH 6 bis 9 verwendet.

2. Verfahren nach Anspruch 1,
**dadurch gekennzeichnet,**
daß als Polymer Polyethylenglykol (PEG) mit einem Molgewicht von 400 bis 20000, insbesondere 1000 bis 4000 verwendet wird.

3. Verfahren nach Anspruch 1 oder 2,
**gekennzeichnet durch**
eine zweistufige Extraktion unter Rückextraktion des zu gewinnenden Proteins aus der primären Oberphase.

4. Verfahren nach Anspruch 3,
**dadurch gekennzeichnet,**
daß für die Rückextraktion NaCl oder PEG mit gegenüber der 1 Stufe erhöhtem Molekulargewicht zugesetzt wird.

5. Verfahren nach einem der vorangehenden Ansprüche,
**dadurch gekennzeichnet,**
daß die Extraktion bei Zimmertemperatur durchgeführt wird.

PEG – Na+ Citrat pH 7,0

FIG. 1

% (w) PEG

MW 400

MW 1550

MW 4000

MW 6000

MW 12000

MW 20000

% (w) Na+ Citrat

EP 0 387 760 A1

FIG 2

PEG 1550 — Na⁺ Citrat pH 7,0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | BIOCHEMICAL AND BIOPHYSICAL ACTA, Band 221, 1970, Seiten 387-390: "Partition of salts and their effects on partition of proteins in a dextran-poly(ethylene glycol)-water two-phase system" * Der ganze Artikel * --- | 1-5 | C 12 N    9/00 C 07 K    3/12 |
| A | TRENDS IN BIOTECHNOLOGY, Band 3, Heft 6, 1985, Seiten 139-144, Elsevier Science Publishers B.V., Amsterdam, NL; H. HUSTEDT et al.: "Protein recovery using two-phase systems" * Der ganze Artikel * --- | 1-5 | |
| A | DE-A-2 639 129 (GESELLSCHAFT FÜR BIOTECHNOLOGISCHE FORSCHUNG) * Seite 47, Beispiele 49-50 * --- | 1-5 | |
| A | EP-A-0 028 016 (GESELLSCHAFT FÜR BIOTECHNOLOGISCHE FORSCHUNG) * Seite 11, Zeilen 5-12 * --- | 1-5 | |
| X | EP-A-0 214 531 (MILES LABORATORIES) * Seiten 9-12; Beispiel 1; Seite 18, Patentansprüche 1-2 * --- | 1-5 | RECHERCHIERTE SACHGEBIETE (Int. Cl.5)  C 12 N C 12 P |
| A | US-A-4 743 550 (KAVSSERY P. ANANTHAPADMANABHAN) * Spalte 4, Zeilen 53-68 * ----- | | |

Der vorliegende Recherchenbericht wurde für alle Patentansprüche erstellt

| Recherchenort | Abschlußdatum der Recherche | Prüfer |
|---|---|---|
| DEN HAAG | 05-06-1990 | FERNANDEZ Y BRANAS F.J. |